# EUROPÄISCHE PATENTSCHRIFT

(11) **EP 1 064 014 B1**
(45) Veröffentlichungstag und Bekanntmachung des Hinweises auf die Patenterteilung: **02.02.2005**
(21) Anmeldenummer: 99916884.2
(22) Anmeldetag: 24.03.1999
(51) Int. Cl.: A61K 38/16, A61K 39/12, A61P 35/00

(54) **ARZNEIMITTEL ZUR VERMEIDUNG ODER BEHANDLUNG VON PAPILLOMAVIRUS-SPEZIFISCHEM TUMOR**
MEDICAMENT FOR PREVENTING OR TREATING PAPILLOMA VIRUS-SPECIFIC TUMORS
MEDICAMENT PERMETTANT DE PREVENIR OU DE TRAITER LES TUMEURS A PAPILLOMAVIRUS

(30) Priorität: 24.03.1998 DE 19812941
(43) Veröffentlichungstag der Anmeldung: 03.01.2001
(73) Patentinhaber: MediGene Aktiengesellschaft, 82152 Martinsried (DE)
(72) Erfinder: BURGER, Alexander, D-55411 Bingen am Rhein (DE); HALLEK, Michael, D-86938 Schondorf (DE)
(74) Vertreter: Bösl, Raphael, Dr. rer. nat., Dipl.-Chem.
(86) Internationale Anmeldenummer: PCT/EP1999/001996
(87) Internationale Veröffentlichungsnummer: WO 1999/048518

(56) Entgegenhaltungen:
- DE-A- 4 435 907
- MÜLLER M ET AL: "Chimeric papillomavirus-like particles" VIROLOGY, Bd. 234, 21. Juli 1997 (1997-07-21), Seiten 93-111, XP002091857 ISSN: 0042-6822 in der Anmeldung erwähnt
- GREENSTONE H L ET AL: "Chimeric papillomavirus virus-like particles elicit antitumor immunity against the E7 oncoprotein in an HPV16 tumor model." PROCEEDINGS OF THE NATIONAL ACADEMY OF SCIENCES OF THE UNITED STATES OF AMERICA, (1998 FEB 17) 95 (4) 1800-5. , XP002117898 in der Anmeldung erwähnt
- DATABASE MEDLINE [Online] US NATIONAL LIBRARY OF MEDICINE (NLM), BETHESDA, MD, US SCHAFER K ET AL: "Immune response to human papillomavirus 16 L1E7 chimeric virus -like particles: induction of cytotoxic T cells and specific tumor protection." retrieved from STN Database accession no. 1999288980 XP002117899 & INTERNATIONAL JOURNAL OF CANCER, (1999 JUN 11) 81 (6) 881-8. ,

## Beschreibung

Die vorliegende Erfindung betrifft die Verwendung mindestens eines Fusionsproteins aus mindestens einem L1-Protein eines oder mehrerer Papillomaviren und mindestens einem C-terminal deletierten E7-Protein eines oder mehrerer Papillomaviren, bei dem 38 bis 43 Aminosäuren deletiert sind, wobei das Fusionsprotein keine Papillomavirus-unspezifischen Epitope enthält, und gegebenenfalls geeignete Zusatz- und/oder Hilfsstoffe zur Herstellung eines Arzneimittels für die Anwendung am Menschen zur Induktion einer spezifischen zytotoxischen T-Zell (CTL)-Antwort einerseits zur Verhinderung von HPV-spezifischen Tumoren und andererseits zur Regression von bereits bestehenden humanen Papillomavirus (HPV)-spezifischen Tumoren.

Die Papillomaviren, auch Warzenviren genannt, sind doppelsträngige DNA-Viren mit einer Genomgröße von etwa 8000 Basenpaaren und einem Ikosaeder-förmigen Kapsid mit einem Durchmesser von ca. 55 nm. Bis heute sind mehr als 100 verschiedene humane Papillomavirustypen bekannt, von denen einige, z.B. HPV-16, HPV-18, HPV-31, HPV-33, HPV-39, HPV-45, HPV-52 oder HPV-58, bösartige Tumore und andere, z.B. HPV-6, HPV-11 oder HPV-42, gutartige Tumore verursachen können.

Elektronenmikroskopische Analysen von BPV-1 und HPV-1 ergaben, daß die Viren aus 72 pentameren Capsomeren aufgebaut sind, die wiederum aus fünf L1-Molekülen bestehen (Baker, T. et al. (1991) Biophys. J., 60, 1445).

Das Genom der Papillomaviren läßt sich in drei Bereiche unterteilen: Der erste Bereich betrifft eine nicht-kodierende Region, die Regulationselemente für die Transkription und Replikation des Virus enthält. Die zweite Region, sogenannte E-(Early)Region enthält verschiedene Protein-kodierende Abschnitte E1-E7, von denen z.B. das E6-Protein und das E7-Protein für die Transformation von Epithelzellen verantwortlich ist und das E1-Protein die DNA-Kopienzahl kontrolliert. Bei der E6- und E7-Region handelt es sich um sogenannte Onkogene, die auch in bösartig entarteten Zellen exprimiert werden. Die dritte Region, auch L-(Late)Region genannt, enthält zwei Protein-kodierende Abschnitte L1 und L2, die für Strukturkomponenten des Viruscapsids kodieren. Das L1-Protein ist zu über 90% im viralen Capsid vorhanden, wobei das Verhältnis von L1:L2 im allgemeinen 30:1 ist.

HPV-6 und HPV-11 werden u.a. für Genitalwarzen verantwortlich gemacht, einige Papillomavirustypen wie HPV-16, HPV-18, HPV-31, HPV-33, HPV-39, HPV-45, HPV-52 und HPV-58 sind mit bösartigen Tumoren des anogenitalen Traktes assoziiert. In über 50% der Fälle wird HPV-16 mit dem Gebärmutterhalskrebs (Cervixcarcinom) in Verbindung gebracht. HPV-16 ist daher der Hauptrisikofaktor für die Ausbildung von cervicalen Neoplasien. Daneben spielt das Immunsystem eine wichtige Rolle beim Fortschritt der Krankheit. So sind vermutlich zelluläre Immunantworten und insbesondere Antigen-spezifische T-Lymphozyten wichtig für den Abwehrmechanismus. Es wurde weiterhin gefunden, daß in hochgradigen cervicalen intraepithelialen Neoplasien (CIN II/III) und cervicalem Tumor das E7-Gen konstitutiv in allen Schichten des infizierten Epithels exprimiert wird. Daher wird das E7-Protein als potentielles Tumorantigen und als Zielmolekül für aktivierte T-Zellen betrachtet (siehe z. B. WO93/20844). Die E7-induzierte zelluläre Immunantwort im Patienten ist aber anscheinend nicht stark genug, um den Krankheitsverlauf zu beeinflussen. Die Immunantwort kann eventuell durch geeignete Impfstoffe verstärkt werden.

Es konnte nun gezeigt werden, daß die Expression des L1-Gens bzw. die Coexpression des L1- und L2-Gens Virus-ähnliche Partikel (VLPs) bildet. Die VLPs konnten zur Bildung von neutralisierenden Antikörpern in verschiedenen tierischen Systemen verwendet werden. Die Bildung von virus-neutralisierenden Antikörpern ist jedoch von geringerer klinischer Bedeutung, wenn die Virusinfektion bereits stattgefunden hat, da für die Eliminierung virus-infizierter Zellen eine virus-spezifische zytotoxische T-Zell(CTL)-Antwort notwendig zu sein scheint. Es wurden daher sogenannte chimäre Papillomavirus-ähnliche Partikel (CVLPs) entwickelt, die aus einem chimären L1-E7-Protein bestehen (Müller, M. et al. (1997) Virology, 234, 93): Einige CVLPs induzieren eine E7-spezifische CTL-Antwort in Mäusen, obwohl Experimente fehlschlugen, Antikörper durch Immunisieren von Mäusen mit CVLPs gegen E7 zu induzieren (Müller, M. et al. (1997), supra). Des weiteren scheinen neutralisierende Antikörper von HPVassoziierten Erkrankungen in Patienten die Immunantwort auf verabreichtes L1-Protein zu limitieren (Müller, M. et al. (1997), supra). CVLPs sind jedoch für die Entwicklung eines Impfstoffes nach wie vor interessant, da die über MHC-Moleküle der Klasse I-präsentierten E7-Proteine von Tumorzellen Zielmoleküle von CTLs darstellen würden.

Peng et al. (1998) Virology, 240, 147 beschreiben nun CVLPs bestehend aus C-terminal verkürztem L1 des Rinderpapillomvirus (BPV) und HPV-16E7₄₉₋₅₇, welche nach Impfung von C57B1/6-Mäusen E7-spezifische zytotoxische T-Zellen induzieren und vor dem Auswachsen E7-exprimierender Tumore schützen. Greenstone et al. (1998) Proc. Natl. Acad. Sci. USA, 95, 1800 beschreiben CVLPs bestehend aus HPV-16L1 plus HPV-16L2 fusioniert mit dem Vollängen HPV-16E7-Protein, welche nach Immunisierung von C57B1/6-Mäusen vor dem Auswachsen epithelialer E7-exprimierender Tumorzellen schützen, wobei jedoch zytotoxische T-Zellen nicht nachgewiesen wurden und somit die Induktion der Immunantwort weniger effizient erscheint.

Die Herstellung von VLPs bzw. CVLPs erfolgt im allgemeinen gentechnisch durch Expression der entsprechenden Gene kodierend für ein oder mehrere L-Proteine bzw. L- und E-Proteine in geeigneten Expressionssystemen. Die entsprechenden Gene sind beispielsweise bei Kirnbaum, R et al. (1994) J. Virol., 67, 6929-6936 beschrieben bzw. über die EMBL-Datenbank erhältlich. Die Zugangsnummern lauten z.B. für HPV18: PAPHPV18; für HPV31: PAPPPH31; für HPV33: PAPPPH33 oder für HPV58: PAPPPH58.

Geeignete Expressionssysteme sind beispielsweise gentechnisch veränderte Hefen, z.B. Saccharomyces (cerevisiae), Pichia (pastoris), Kluyvermyces (lactis), Schizosaccharomyces (pombe) oder Hansenula (polymorpha) (Carter, J. J. et al. (1991), Virology, 182, 513), Insektenzellen, wie z.B. Trichoplusia ni High Five (siehe z.B. Müller et al. (1997), supra) oder prokaryotische Zellen (siehe z.B. WO 96/11272). Bei der Herstellung der Partikel in prokaryotischen Zellen fallen diese im allgemeinen in der Zelle aus und bilden sogenannte Einschlußkörperchen (Inclusion Bodies), die anschließend renaturiert und in Lösung gebracht werden müssen. Für die Verwendung der gentechnisch hergestellten Partikel bzw. Capside oder deren Vorstufen, den sogenannten Capsomeren, sind nach der Expression weitere Reinigungsschritte notwendig.

Ein entscheidender Nachteil der in der Literatur beschriebenen Wirkstoffe gegen HPV ist jedoch, daß sie zum einen nur eine geringe Wirkung zeigen und zum anderen bis heute keine wirksame Immuntherapie von Papillomavirus-spezifischem Tumor gezeigt werden konnte.

Aufgabe der vorliegenden Erfindung war es daher, ein Arzneimittel bereitzustellen, mit dem wirksam humaner Papillomavirus-spezifischer Tumor vermieden bzw. behandelt werden kann, das einfach hergestellt werden kann und das für die Zulassung als Arzneimittel geeignet erscheint.

Es wurde nun überraschenderweise gefunden, daß das beschriebene Arzneimittel gegen HPV-spezifischen Tumor wirksam ist.

Gegenstand der vorliegenden Erfindung ist daher die Verwendung mindestens eines Fusionsproteins aus mindestens einem L1-Protein eines oder mehrerer Papillomaviren und mindestens einem C-terminal deletierten E7-Protein eines oder mehrerer Papillomaviren, bei dem 38 bis 43 Aminosäuren deletiert sind, wobei das Fusionsprotein keine Papillomavirus-unspezifischen Epitope enthält, und gegebenenfalls geeignete Zusatz- und/oder Hilfsstoffe zur Herstellung eines Arzneimittels für die Anwendung am Menschen zur Induktion einer spezifischen zytotoxischen T-Zell (CTL)-Antwort einerseits zur Verhinderung von HPV-spezifischen Tumoren und andererseits zur Regression von bereits bestehenden humanen Papillomavirus (HPV)-spezifischen Tumoren.

Unter Papillomavirus-unspezifische Epitope im Sinne der vorliegenden Erfindung versteht man im allgemeinen Epitope im Fusionsprotein, die durch einen Fremdproteinanteil, durch post-translationale Modifikationen oder durch eine Fehlfaltung der Papillomavirus-spezifischen Proteine verursacht werden.

Die Papillomavirus-unspezifischen Epitope sind beispielsweise eine Ursache dafür, daß zwar neutralisierende Antikörper oder CTL-Immunantworten induziert werden, jedoch ein Papillomavirus-spezifischer Tumor nicht wirksam vermieden bzw. bekämpft werden kann, da durch unspezifische Antikörper oder CTLs die immunologische Wirkung abgeschwächt wird oder immunologische Nebenwirkungen die Wirkung des eigentlichen Wirkstoffes stören.

Das beschriebene Arzneimittel wirkt vorzugsweise zur Vermeidung oder Behandlung von gutartigem oder bösartigem Tumor, insbesondere von Larynx-, Cervix-, Penis-, Vulva- oder Analcarcinom, einschließlich deren Vorstufen, wie z.B. hochgradige CIN (cervikale intraepitheliale Neoplasie).

In einer weiteren bevorzugten Ausführungsform enthält das beschriebene Arzneimittel kein Adjuvans, d.h. keine Substanz, die die Immunität des Papillomavirus-spezifischen Proteins verstärkt, da insbesondere die Anwesenheit eines L-Proteins vor allem von L1 die Immunität bereits ausreichend verstärkt. Diese Eigenschaft ist insbesondere bei der Zulassung als Arzneimittel oder Diagnostikum von Vorteil, da die einzigen von den Zulassungsbehörden zugelassenen immunstimulierenden Materialien zur Zeit Aluminiumsalze sind. Zudem werden durch das Weglassen von Adjuvantien und/oder anderen Hilfs- bzw. Zusatzstoffen nicht erwünschte Nebenwirkungen vermieden.

Wie bereits oben erwähnt ist ein weiteres wesentliches Problem bei der Verwendung von Capsiden und Capsomeren als Arzneimittel deren schlechte Löslichkeit. So neigen beispielsweise Capside oder Capsomere von HPV-16 zur Aggregation, wodurch die Löslichkeit wesentlich verringert wird. Die zum Teil geringe Löslichkeit der Capside bzw. Capsomere führt nicht nur zu einem Verlust an Ausbeute, sondern auch zu einer erschwerten Anwendung als Arzneimittel.

In einer weiteren bevorzugten Ausführungsforrn enthält das beschriebene Arzneimittel daher als einen geeigneten Zusatz- oder Hilfsstoff 0,3 bis 4 M, vorzugsweise 0,4 bis 3 M, insbesondere 0,5 bis 2 M, vor allem 1 bis 2 M eines Salzes mit einem pH-Wert von 7,3 bis 7,45, vorzugsweise 7,4.

Der Vorteil dieser Salzlösung ist, daß das Fusionsprotein in Lösung bleibt bzw. fein verteilt als Suspension vorliegt, d.h. es bleiben im allgemeinen mehr als ca. 90%, vor allem mehr als ca. 95% des Fusionsproteins in Lösung und fallen auch nicht für einen Zeitraum von mindestens ca. 12 Stunden aus. Auch ist das Fusionsprotein durch Zentrifugation bei maximal 5000 g nicht wesentlich sedimentierbar.

Das Salz ist im allgemeinen ein Alkali- oder Erdalkalisalz, vorzugsweise ein Halogenid oder Phosphat, insbesondere ein Alkalihalogenid, vor allem NaCl und/oder KCI. Die Verwendung von NaCl ist insbesondere für die Herstellung einer pharmazeutischen Formulierung bevorzugt.

Der pH-Wert des Arzneimittels wird im allgemeinen mit einem geeigneten organischen oder anorganischen Puffer eingestellt, wie z.B. vorzugsweise mit einem Phosphat-Puffer, Tris-Puffer (Tris(hydroxymethyl)-aminomethan), HEPES-Puffer ([4-(2-Hydroxyethyl)-piperazino)-ethansulfonsäure) oder MOPS-Puffer (3-Morpholino-1-propansulfonsäure). Die Auswahl des jeweiligen Puffers richtet sich im allgemeinen nach der gewünschten Puffermolarität. Phosphatpuffer ist beispielsweise für Injektions- und Infusionslösungen geeignet.

Als weitere Zusatz- und/oder Hilfsstoffe, die beispielsweise zur weiteren Stabilisierung des Papillomavirus-spezifischen Proteins in dem erfindungsgemäßen Arzneimittel dienen, eignen sich beispielsweise Detergentien, wie z.B. Triton-X-100 oder Natriumdesoxycholat, aber auch Polyole, wie z. B. Polyethylenglykol oder Glycerin, Zucker, wie z. B. Saccharose oder Glukose, zwitterionische Verbindungen, wie z. B. Aminosäuren wie Glycin oder insbesondere Taurin oder Betain und/oder ein Protein, wie z. B. bovines oder humanes Serumalbumin. Bevorzugt sind Detergentien, Polyole und/oder zwitterionische Verbindungen. Andere Zusatz- und/oder Hilfsstoffe sind Proteaseinhibitoren, wie z.B. Aprotinin, ε-Aminocapronsäure oder Pepstatin A. Bevorzugt sind solche Zusatzstoffe, die keine immunologischen Nebenwirkungen induzieren.

Unter den Begriffen L1/L2-Protein und E-Protein versteht man im Sinne der vorliegenden Erfindung sowohl die Vollängen-Proteine wie auch deren Mutanten, wie z. B. Deletionsmutanten.

In einer weiteren bevorzugten Ausführungsform enthält das beschriebene Fusionsprotein ein deletiertes L1-Protein. Die Deletion hat den Vorteil, daß in den deletierten Bereich besonders wirksam andere Proteine, beispielsweise Papillomavirus-spezifische E-Proteinsequenzen eingefügt werden können, wodurch sich der Anwendungsbereich der erfindungsgemäßen Zusammensetzung erweitern läßt. Insbesondere bevorzugt ist ein L-Protein mit einer C-terminalen Deletion und insbesondere ein C-terminal deletiertes L1-Protein. Die C-terminale Deletion hat den Vorteil, daß die Effizienz der Bildung virus-ähnlicher Partikel gesteigert werden kann, da das am C-Terminus lokalisierte nukleäre Lokalisationssignal deletiert ist. Die C-terminale Deletion beträgt daher vorzugsweise bis zu 35 Aminosäuren, insbesondere 25 bis ca. 35 Aminosäuren, vor allem 32 bis 34 Aminosäuren. Beispielsweise ist eine 32 Aminosäuren lange C-terminale Deletion des HPV-16 L1-Proteins ausreichend, um die Bildung von virusähnlichen Partikeln um das mindestens ca. zehnfache steigern zu können.

In der erfindungsgemäßen Ausführungsform ist auch das E7-Protein deletiert, da diese Konstrukte in Verbindung mit deletiertem L-Protein bevorzugt Capsomere und/oder Capside ausbilden können.

Ein besonders bevorzugtes Konstrukt ist beispielsweise E7 mit den N-terrninalen Aminosäuren 1 bis 60, da dieses Konstrukt ein Maus-Epitop zur Aktivierung von zytotoxischen T-Lymphozyten enthält, welches im Bereich der Aminosäuren 49-57 lokalisiert ist. Ein anderes bevorzugtes Konstrukt ist E7 mit den N-terminalen Aminosäuren 1 bis 55, welches in Verbindung mit deletiertem L-Protein Capsomere und Capside bevorzugt bildet, da dieses Konstrukt E7-spezifische Sequenzen im Bereich der Aminosäuren 56-70 nicht enthält, welche die Bildung von Capsiden stören können. Besonders bevorzugt ist ein um 32 Aminosäuren C-terminal deletiertes L1-Protein von HPV-16, welches mit einem E7-Protein von HPV-16 mit den Aminosäuren 1-55 oder 1-60 verbunden ist. Diese Konstrukte induzieren nicht nur neutralisierende Antikörper oder eine spezifische CTL-Antwort, sondern verhindert einerseits die Bildung von Tumoren und verursachen andererseits einen Rückgang von bereits bestehenden Tumoren im Tierexperiment. Vor allem E7 mit den Aminosäuren 1-60 zeigt eine deutliche prophylaktische wie auch therapeutische Wirkung bei Tumoren. Eine besonders bevorzugte Ausführungsform der vorliegenden Erfindung ist daher ein L1ΔCE7₁₋ₓ-Fusionsprotein, vorzugsweise in Form eines CVLPₛ, insbesondere von HPV16, wobei x eine ganze Zahl von 55 bis einschließlich 60 bedeutet, und insbesondere ein L1ΔCE7₁₋₅₅- oder LIACE7₁₋₆₀-Fusionsprotein.

Zur Herstellung eines sowohl prophylaktisch wie auch therapeutisch wirksamen Arzneimittels ist es bevorzugt, wenn das beschriebene Papillomavirus-spezifische Fusionsprotein in Form eines Capsids und/oder Capsomers vorliegt, da durch die Capside und/oder Capsomere und insbesondere durch den Anteil von L-Protein die Immunreaktion noch deutlich gesteigert werden kann. Als Fusionsproteine, die zur Capsid- und/oder Capsomerbildung geeignet sind, sind daher beispielsweise Fusionsproteine aus deletiertem L1 und E7 bevorzugt.

Capside sind im Sinne der vorliegenden Erfindung virale oder virus-ähnliche Strukturen in einer im allgemeinen ikosaedrischen Form, die im allgemeinen aus 72 Capsomeren aufgebaut sind.

Capsomere sind im Sinne der vorliegenden Erfindung assemblierte Proteine enthaltend mindestens ein Papillomavirus-Strukturprotein, vorzugsweise L1 oder Deletionen von L1. Beispielsweise können 5 erfindungsgemäße Fusionsproieine zu einem Capsomer assemblieren, die wiederum zu einem Capsid assemblieren können.

Für die Herstellung eines humanen Arzneimittels sind für die beschriebenen Konstrukte Proteine bzw. Peptide des humanen Papillomavirus (HPV) und vorzugsweise von HPV-6, BPV- 11, HPV-16, HPV-18, HPV-31, HPV-33, HPV-35, HPV-39, HPV-45, HPV-52 und/oder HPV-58, insbesondere HPV-16, HPV-18, HPV-31 und/oder HPV-45 geeignet. Vor allem für die Herstellung einer KombinationsVakzine ist es vorteilhaft, Proteine bzw. Peptide aus verschiedenen HPV-Typen zu kombinieren, beispielsweise eine Kombination von HPV-16 und HPV-18 bzw. HPV-18, HPV-31, HPV-45 und HPV-58 im Falle von z. B. Cervixcarcinom oder HPV-6 und HPV-11 im Fall von z. B. Condylomen.

Bei dem Verfahren zur Herstellung eines erfindungsgemäßen Arzneimittels wird eine geeignete Zelle enthaltend einen geeigneten Expressionsvektor, der fürdas genannte Fusionsprotein kodiert, unter geeigneten Bedingungen kultiviert, das Expressionsprodukt isoliert und gegebenenfalls geeignete Zusatz- und/oder Hilfsstoffe zugesetzt.

Die Expressionsvektoren können beispielsweise prokaryotische oder eukaryotische Expressionsvektoren sein. Beispiele für prokaryotische Expressionsvektoren sind für die Expression in E. coli z.B. die Vektoren pGEM oder pUC-Derviate (siehe z. B. WO96/11272). Beispiele für eukaryotische Expressionsvektoren sind für die Expression in Saccharomyces cerevisiae z. B. die Vektoren p426Met25. oder p426GAL1 (Mumberg et al. (1994) Nucl. Acids Res., 22, 5767-5768, Carter, J. J. et al. (1991) supra) und für die Expression in Insektenzellen z. B. Baculovirus-Vektoren, insbesondere das Autographa Californica-Virus, wie in EP-B1-0 127 839 oder EP-B 1-0 549 721 offenbart (siehe z. B. auch WO94/20137), und für die Expression in Säugerzellen z. B. die Vektoren Rc/CMV und Re/RSV oder SV40-Vektoren, welche alle allgemein erhältlich sind. Es eignen sich jedoch auch kommerziell erhältliche Baculovirus Expressionssysteme wie z. B. der Baculo Gold ^{TM} Transfection Kit von Pharmingen oder das Bac-to-Bac^{TM} Baculovirus Expressionsystems von Gibco BRL. Weitere geeignete Expressionssysteme sind rekombinante Vaccinia-Viren (siehe z. B. WO 93/02184)

Im allgemeinen enthalten die Expressionsvektoren auch für die jeweilige Wirtszelle geeignete Promotoren, wie z. B. den trp-Promotor für die Expression in E. coli (siehe z. B. EP-B1-0 154 133), den ADH2-Promotor für die Expression in Hefen (Russel et al. (1983), J. Biol. Chem. 258, 2674-2682), den Baculovirus-Polyhedrin-Promotor für die Expression in Insektenzellen (siehe z. B. EP-B 1-0 127 839 oder U.S. 5,004,687) oder den frühen SV40 Promotor oder LTR-Promotoren z. B. von MMTV (mouse mammary tumour virus; Lee et al. (1981) Nature 214, 228-232).

Als Wirtszellen eignen sich beispielsweise die E. coli Stämme DH5, HB101 oder BL21, die Hefestämme Saccharomyces, Pichia, Kluyvermyces, Schizosaccharomyces oder Hansenula (Carter, J. J. et al. (1991), Virology, 182, 513), die Insektenzellinie Lepidopteran, z. B. von Spodoptera frugiperda, Trichoplusia ni, Rachiplusia ou oder Galleria Mellonela oder die tierischen Zellen COS, C127, Vero, 293 und HeLa, die alle allgemein erhältlich sind (siehe z. B. WO94/00152).

Die kodierenden Nukleinsäuren für die einzelnen Papillomavirus-spezifischen Proteine können beispielsweise über eine PCR("polymerase chain reaction")-Amplifizierung aus einer Genbank isoliert und kloniert werden. Beispielsweise ist das Genom von BPV-1 unter der GenBank Accession No. X02346 oder HPV-16 unter der GenBank Accession No. K02718 allgemein erhältlich. Eine HPV-16 L1-Sequenz ist beispielsweise auch in WO94/05792 offenbart. Die Sequenz des 98 Aminosäure-langen HPV16 E7-Proteins ist beispielsweise bei Seedorf et al. (1985) Virology, 145, 181-185 beschrieben. Eine andere Methode, die gewünschten Nukleinsäuren zu erhalten, ist, die Papiflomavirus-spezifischen Gene direkt aus Warzen oder Tumore mittels PCR zu isolieren. Geeignete Primer für die E6- und E7-Gene aus HPV-16 und HPV-18 sind z. B. in WO93/21958 offenbart. Weitere Literaturstellen für die gewünschten Nukleinsäuren sind beispielsweise Kirnbaum, R. et al. (1994), supra bzw. die oben bereits erwähnten in der EMBL-Datenbank hinterlegten Klone.

In einer weiteren bevorzugten Ausführungsform ist der Expressionsvektor so konstruiert, daß das exprimierte Fusionsprotein um keine weiteren, durch den Vektor verursachte Aminosäuren verlängert ist. Dies wird beispielsweise dadurch erreicht, daß durch Mutagenese in einer PCR-Reaktion mittels geeigneter Primer-Oligonucleotide unerwünschte Nucleotide, die für zusätzliche Aminosäuren kodieren, entfernt werden (Ho et a. (1989) Gene, 77, 51-59). Auf diese Weise erhält man ein Fusionsprotein, welches frei von zusätzlichen Aminosäuren und somit frei von möglicherweise zusätzlichen Fremdepitopen ist, die immunologische Nebenreaktionen bewirken können.

Nach der Expression des beschriebenen Fusionsproteins ist es bevorzugt, dieses weiter zu reinigen bzw. zu renaturieren. Beispiele von chromatographischen Reinigungsverfahren finden sich Hjorth, R. & Moreno-Lopez, L. (1982) J. Virol. Meth., 5, 151; Nakai, Y. et al. (1987) J. Gen. Virol., 68, 1891; Hofmann, K. J. et al. (1995) Virology, 209, 506; Rose, R. C. et al. (1993) J. Virol., 67, 1936, Sasagawa, T. et al. (1995) Virology, 206, 126 oder WO 95/31532.

Im allgemeinen kann das Arzneimittel oral, parenteral, wie z.B. subcutan, intramuskulär oder über die Schleimhaut, in flüssiger oder suspendierter Form, in Form eines Elixiers oder als Kapseln verabreicht werden, vorzugsweise als Injektions- bzw. Infusionslösung. Bei den beschriebenen Formulierungen kann auf ein Adjuvans verzichtet werden, was besonders vorteilhaft ist.

Ein weiterer Gegenstand der vorliegenden Erfindung bezieht sich daher auf die Verwendung der beschriebenen Formulierung als Injektions- oder Infusionslösung.

Injektionslösungen werden im allgemeinen dann verwendet, wenn nur relativ geringe Mengen einer Lösung bzw. Suspension, beispielsweise ca. 1 bis ca. 20 ml, dem Organismus zugeführt werden soll. Infusionslösungen werden im allgemeinen dann verwendet, wenn eine größere Menge einer Lösung oder Suspension, beispielsweise ein oder mehre Liter, appliziert werden sollen. Da im Gegensatz zur Infusionslösung bei Injektionslösungen nur wenige Milliliter verabreicht werden, machen sich geringe Abweichungen vom pH-Wert und vom osmotischen Druck des Blutes bzw. der Gewebsflüssigkeit bei der Injektion nicht oder nur unwesentlich im Hinblick auf die Schmerzempfindung bemerkbar. Eine Verdünung der erfindungsgemäßen Formulierung vor der Anwendung ist daher im allgemeinen nicht erforderlich. Bei der Applikation größerer Mengen sollte hingegen kurz vor der Applikation die erfindungsgemäße Formulierung so weit verdünnt werden, daß eine zumindest annähernd isotonische Lösung erhalten wird. Ein Beispiel einer isotonischen Lösung ist eine 0,9%-ige Natriumchloridlösung. Bei der Infusion kann die Verdünnung beispielsweise mit sterilem Wasser während der Applikation z.B. über einen sog. Bypaß erfolgen.

Die Figuren und die folgenden Beispiele sollen die Erfindung näher erläutern, ohne sie zu beschränken.

### Beschreibung der Figuren

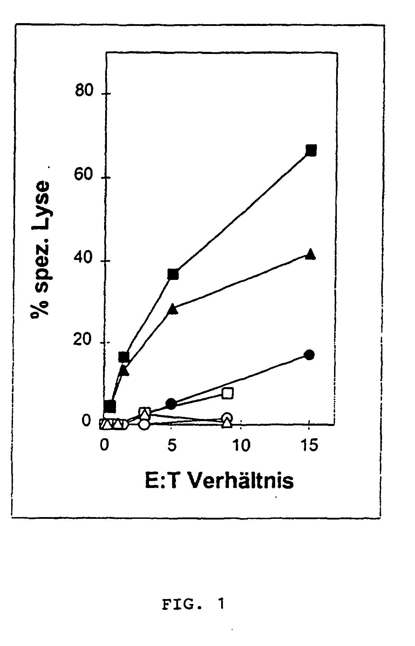

Fig. 1 zeigt eine E7-spezifische CTL-Antwort, die nach der Immunisierung mit 1 µg L1E7₁₋₆₀ CVLPs erzeugt wurden. Die isolierten Milzzellen von mit L1E7₁₋₆₀ CVLPs (gefüllte Zeichen) und HBS-Puffer (offene Zeichen) immunisierten Mäusen wurden einmal in vitro mit bestrahlten RMA-E7-Zellen stimuliert und nach fünf Tagen in einem Standard vier Stunden ⁵¹Cr-Freisetzungszytotoxizitätstest mit folgenden Zielzellen getestet: RMA-E7-Zellen (Quadrate), E7₄₉₋₅₇ Peptid-beladene RMA-Zellen (dreieckig) und RMA-Zellen (Kreise). Die Ergebnisse sind als spezifische Lyse in % ausgedrückt.

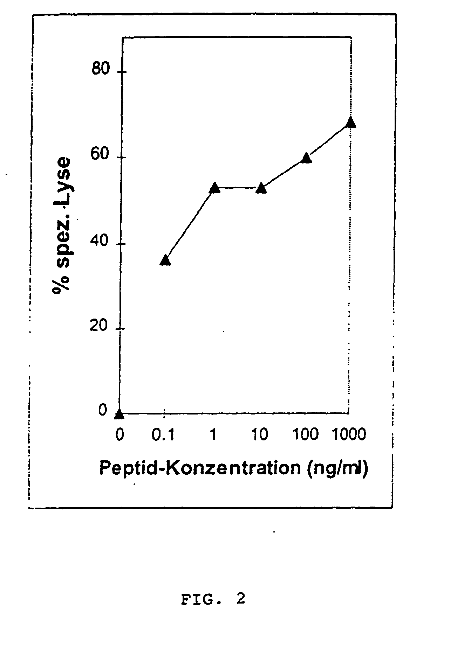

Fig. 2 zeigt das Ergebnis eines Titrationstestes, durchgeführt mit einer E7-spezifischen CTL-Linie, die nach Vaccinierung einer C57BL/6-Maus mit 20 µg L1E7₁₋₆₀ CVLPs und dreimaliger in vitro Stimulation mit RMA-E7 Transfektanten erhalten wurde. Die Peptidkonzentration lag zwischen 100 pg und 1 µg/ml. Als Zielzellen wurden ⁵¹Cr-markierte E7 49-57 Peptid-beladene RMA-Zellen verwendet. Das Verhältnis von Zellen zu Zielzellen war 30:1.

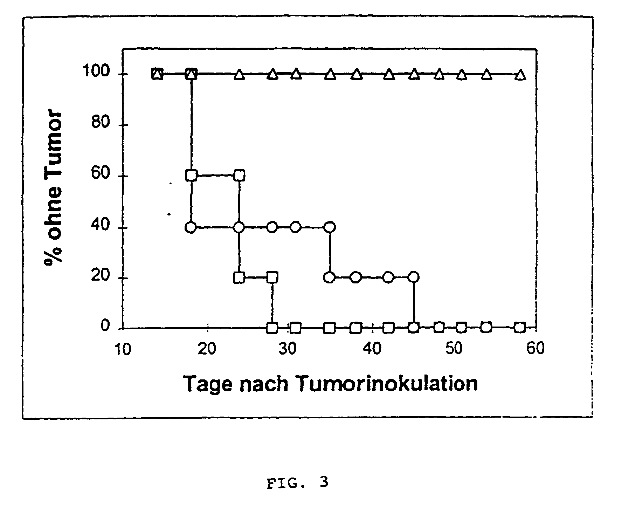

Fig. 3 zeigt den Schutz von C57BL/6-Mäusen gegen das Wachstum von TC-1 Tumorzellen. Die Mäuse (5 pro Gruppe) wurden s.c. entweder mit 10 µg L1E7₁₋₆₀ CVLPs (dreieckig), mit 10 µg L1ΔC VLPs (Kreise) oder mit HBS-Puffer (Quadrate) immunisiert. Zwei Wochen später wurden den Mäusen s.c. 6 x 10⁴ TC-1 Tumorzellen pro Maus in die linke Flanke inokuliert. Die Mäuse wurden zweimal pro Woche kontrolliert.

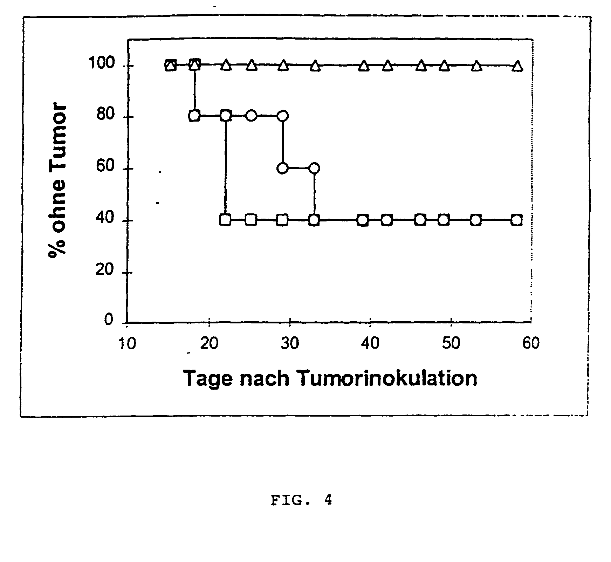

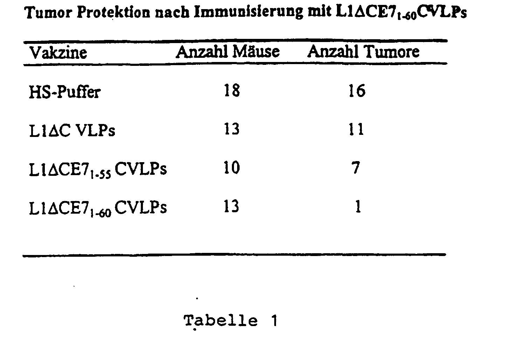

Fig. 4 zeigt die Verhinderung des Wachstums von TC-1 Tumor durch L1E7₁₋₆₀ CVLPs. C57BL/6-Mäusen (5 pro Gruppe) wurden 6 x 10⁴ TC-1-Tumorzellen pro Maus in die linke Flanke inokuliert. Zwei Wochen später wurden die Mäuse mit einer s.c. Injektion von 10 µg L1E7₁₋₆₀ CVLPs (Dreiecke), 10 µg L1ΔCVLPs (Kreise) oder HBS-Puffer (Quadrate) immunisiert.

### Beispiele

### 1. Herstellung von chimären Genen kodierend für HPV16L1E7-Fusionsproteine

Der HPV-16L1 offene Leserahmen (ORF) wurde aus dem Plasmid HPV-16-114/k-L1/L2-pSynxtVI⁻ (Kirnbauer, R et al. (1994) J. Virol. 67, 6929) mit der Restriktionsendonuklease BglII ausgeschnitten und in den Vektor pUC19 (New England Biolabs) in die BamHI-Stelle kloniert.

Zur Herstellung von HPV-16L1ΔC wurden zwei Primer konstruiert, die zu HPV-16L1 ORF komplementär sind. Der erste Primer hat die Sequenz AAAGATATCTTGTAGTAAAAATTTGCGTCCTAAAGGAAAC und der zweite Primer

AAAGATATCTAATCTACCTCTACAACTGCTAAACGCAAAAAACG.

Beide Primer kodieren 5' eine EcoRV Restriktionsenzymschnittstelle. In den stromabwärtsliegenden Primern folgt auf die EcoRV Stelle ein TAA Translationsstopkodon, um die letzten 34 Aminosäuren des HPV16L1 ORF zu deletieren. Die PCR-Reaktion wurde durchgeführt, um den gesamten L1 ORF und den gesamten Vektor zu amplifizieren. Das lineare Produkt wurde mit EcoRV gespalten, mit T4 DNA-Ligase zirkularisiert und E.coli DH5α-Zellen transformiert. Die Klone wurden auf die Anwesenheit einer EcoRV Stelle analysiert. Das erhaltene Konstrukt pUCHPV16L1ΔC wurde benutzt, um den ORF von HPV16E7 1-50 in die EcoRV Stelle zu klonieren.

Zur Klonierung des Fragmentes wurden Primer mit einer 5'EcoRV Restriktionsenzymschnittstelle verwendet. Es wurde folgendes Primerpaar verwendet: AAAAGATATCATGCATGGAGATACACCTACATTGC und

TTTTGATATCGGCTCTGTCCGGTTCTGCTTGTCC.

Die PCR Produkte wurden mit EcoRV gespalten und in die EcoRV Stelle des modifizierten L1-Gens insertiert.

Zur Eliminierung der EcoRV Stellen wurden zwei PCR-Reaktionen durchgeführt, um zwei überlappende Fragmente des Klons pUC-HPV16L1ΔCE7 1-50 zu amplifizieren. Die resultierenden DNA-Fragmente überlappten an der Position der L1/E7 Grenze ("Four Primer PCR", Ho, S. N. et al (1989) Gene 77, 51). Jedoch enthielten die Primer nicht die zwei EcoRV Restriktionsenzymschnittstellen. Fragment 1 wurde mit den Primern P 1 und P2 und Fragment 2 mit den Primern P3 und P4 hergestellt.

P1: GTTATGACATACATACATTCTATG (L1)
P2: CCATGCATTCCTGCTTGTAGTAAAAATTTGCGTCC (E7)
P3: CTACAAGCAGGAATGCATGGAGATACACC (E7)
P4:CATCTGAAGCTTAGTAATGGGCTCTGTCCGGTTCTG (E7)

Ein Zehntel der gereinigten Produkte wurde gemischt und als Matrize in der PCR-Reaktion mit ausschließlich den Primern P1 und P4 benutzt. Das resultierende Produkt wurde mit EcoNI (L1) und HindIII (stromabwärts vom Stopkodon auf den Primer P4) gespalten und benutzt, um ein EcoNI/HindIII Fragment des klonierten HPV16L1 ORF zu ersetzen. Der resultierende Klon unterscheidet sich daher vom Klon HPV16L1ΔCE7 1-50 durch den Verlust der zwei internen EcoRV Restriktionsenzymschnittstellen und der korrespondierenden nicht-HPV Aminosäuren Asp und Ile zwischen dem L1 ORF und E7 und stromabwärts von E7. Die erste EcoRV Stelle wurde durch die ursprünglichen L1 Aminosäuren an dieser Position ersetzt (AlaGly). Die zweite EcoRV Stelle wurde durch ein Translationsstopsignal ersetzt. Zusätzlich enthält dieser Klon (HPV16L1ΔC*E7 1-52) die ersten 52 Aminosäuren von HPV16E7. Klon HPV16L1ΔC*E7 1-52 wurde zur Herstellung der Klone HPV16L1ΔC*E7 1-55, 1-60 und 1-65 mit Hilfe des Primers P1 in Kombination mit P5, P6 und P7 verwendet.

P5: CATCTGAAGCTTATCAATATTGTAATGGGCTCTGTCCG (E7 1-55)
P6:
CATCTGAAGCTTACTTGCAACAAAAGGTTACAATATTGTAATGGGCTCT
GTCCG (E7 1-60)
P7:
CATCTGAAGCTTAAAGCGTAGAGTCACACTTGCAACAAAAGGTTACAA
TATTGTAATGGGCTCTGTCCG (E7 1-65).

HPV16L1ΔC*E7 1-70 wurde mit dem Klon HPV16L1ΔC*E7 1-65 und den Primern P1 und P8 hergestellt.

P8:
CATCTGAAGCTTATTGTACGCACAACCGAAGCGTAGAGTCACACTTG

In allen Fällen wurden EcoNI und HindIII benutzt, um die korrespondierenden Fragmente zu ersetzen. Die Klone wurden durch DNA-Sequenzierung analysiert.

### 2. Herstellung, von rekombinanten Baculoviren

Spodoptera frugiperda (Sf9) Zellen wurden als Monolayer oder in Suspensionskultur in TNM-FH Insektenmedium (Sigma, Deisenhofen) mit 10% fötalem Kälberserum und 2 mM Glutamin herangezogen. Rekombinante Baculoviren HPV16L1ΔCE7 1-x wurden durch Cotransfektion von 10 µg der rekombinanten Plasmide und 2 µg von linearisiertem Baculo-Gold DNA (Pharmingen, San Diego, CA) in Sf9 Zellen transfiziert. Rekombinante Viren wurden gemäß der Angaben des Herstellers gereinigt. Um die Expression zu testen, wurden 10⁶ Sf9 Zellen mit rekombinantem Baculovirus und einer m.o.i. ("multiplicity of infection") von 5 bis 10 infiziert. Nach der Inkubation wurde das Medium entfernt und die Zellen mit PBS (140 mM NaCl, 2,7 mM Kcl, 8,1 mM Na₂PO₄, 1,5 mM KH₂O₄, pH 7,2) gewaschen. Die Zellen wurden dann in SDS-Probenpuffer lysiert und durch SDS-Gelchromatographie und Immunoblotassay getestet.

### 3. Reinigung von virusähnlichen Partikeln

Für die Herstellung von CVLPs wurden Trichoplusia ni (TN) High Five-Zellen bei 27°C bis zu einer Dichte von 1-1,5 x 10⁶ Zellen pro ml in Ex-Cell 405 serumfreiem Medium gezüchtet (JRH, Biosciences, Lennexa, KS). Eine 400 ml Kultur wurde geerntet und mit einer m.o.i. von 2 bis 5 mit rekombinanten Baculoviren für eine Stunde mit periodischen Inversionen infiziert. Es wurden bis zu 240 ml Medium hinzugegeben und die Zellen wuchsen 3 bis 4 Tage lang. Anschließend wurden die Zellen pelletiert und in 10 ml Extraktionspuffer resuspendiert (10 mM MgCl₂, 1-50 mM CaCl₂, 150 mM NaCl, 20 mM Hepes, 0,01% Triton (optional), pH 7,4) und für 45 Sekunden bei 60 Watt beschallt. Nach der Zentrifugation bei 10.000 rpm im Sorvall SS34 Rotor wurde das Pellet im 6 ml Extraktionspuffer gelöst, für 30 Sekunden bei 60 Watt beschallt und erneut zentrifugiert. Die Überstände wurden kombiniert und auf einen Zweistufengradienten aus 40% (w/v) Succhrose und 57,5% (w/v) CsCl aufgebracht. Nach der Zentrifugation in einem SW-28 Rotor bei 27.000 rpm für zwei Stunden wurden die Interphase und die CsCl-Schicht gesammelt, auf eine CsCI-Dichte von 1,38 g/ml justiert und bei 45.000 rpm für 16 Stunden zentrifugiert. Die Gradienten wurden fraktioniert und jede Fraktion wurde durch Western Plot mit Anti-HPV16L1mAb Camvir1 (Pharningen, San Diego, CA) getestet. Die reaktiven Fraktionen wurden vereinigt und über eine Ultrafiltration mit Centricon 30 Mikrokonzentrator (Amicon Corp. Beverly, MA) gegen Hepes-Puffer (1 mM Hepes, 149 mM NaCl, 0,5 mM KCl, pH 7,2) dialysiert und die Anwesenheit von CVLPs mittels Transmissions-Elektronenmikroskopie bestätigt. Die Konzentration von L1E7-Protein wurde in einem SDS-Gel, welches mit Coomassie blue angefärbt wurde, durch Vergleich mit BSA-Standards ungefähr bestimmt.

### 4. Kultivierung von Mauszellen

Die C57BL/6 abgeleiteten Mauszellen TC-1 sind primäre Lungenepithelzellen von C57BL/6-Mäusen, die durch Transfektion mit den Onkogenen HPV-16 E6 und E7 und c-Ha-ras (Lin , K.-Y. (1996), supra) transformiert wurden. RMA (Ljungrenn & Karre (1985) J. Exp. Med., 162, 1745-1757) und die prozessierungsdefekte Zellinie RMA-S sind C57BL/6 Thymoma-Zellen. Die HPV-16 E7 transfizierte RMA-E7 ist in Speidel, K. et al. (1997) Eur. J. Immunol., 27(9), 2391-2399, beschrieben. Alle Zellen wurden in RPMI-1640, supplementiert mit 10% FCS, 2-ME, L-Glutamin und Antibiotika, kultiviert. 0,8 mg/ml G418 (Gibco BRL, Gaithersburg, MD) wurde für die Kultivierung von RMA-E7-Transfektanten hinzugegeben. TC-1-Zellen wuchsen zusätzlich in Anwesenheit von 0,4 mg/ml G418, 0,2 mg/ml Hygromycin und 1 mM Natriumpyruvat.

### 5. Herstellung von cytotoxischen T-Zell(CTL)-Linien

10-14 Tage nach der Immunisierung wurden die Milzzellen präpariert, 2-4x10⁷ Milzzellen wurden mit 10⁵ bestrahlten (200 Gy) syngenen RMA-E7-Transfektanten pro ml cokultiviert. Nach 5 Tagen wurde der erste ⁵¹Cr-Freisetzungszytotoxizitätstest durchgeführt. Für die Herstellung von CTL-Linien wurden die Milzzellen wöchentlich in 24-Lochplatten mit 2x10⁵ bestrahlten RMA-E7-Transfektanten pro Loch als Stimulatorzellen und 5x10⁶ bestrahlten (33 Gy) C57BL/6 Milzzellen pro Loch als Feederzellen restimuliert.

### 6. ⁵¹Cr-Freisetzungscytotoxizitätstest

Für den ⁵¹Cr-Freisetzungscytotoxizitätstest wurden Effektor-T-Zellen zu 1x10⁴ ⁵¹Cr-markierten Zielzellen pro Loch auf einer 96 Lochplatte mit unterschiedlichen Effektorzell zu Zielzellverhältnis (E:T) hinzugegeben. Die Zielzellen wurden mit Na₂⁵¹CrO₄ (100 µCi pro 2 x 10⁶ Zellen) für eine Stunde bei 37°C markiert. Das Peptid mit der Sequenz RAHYNIVTF (Aminosäure 49-57 von HPV-16 E7; Feltkamp, M.C.W. et al. (1993), Eur. J. Immunol., 23, 2242-2249) wurde während dieser Inkubation in einer Konzentration von 50 µM hinzugegeben. In dem Peptidtitrationsstest mit einem konstanten E:T-Verhältnis wurden die ⁵¹Crmarkierten Zielzellen mit abnehmenden Peptidkonzentrationen (1 µg - 100 pg pro ml) für eine Stunde bei 37°C kultiviert bevor die Effektorzellen hinzugegeben wurden. Nach vierstündiger Inkubation bei 37°C, wurden 50 µl des Überstandes pro Loch auf eine Lumaplatte (Packard) transferiert und getrocknet. Die Radioaktivität wurde in einem ß-Zähler (Trilux Microbeta, Wallac) gemessen. Die mittlere spezifische Lyse wurde gemäß folgender Formel berechnet: % spezifische Lyse = (experimentelle Freisetzung - spontane Freisetzung)/(vollständige Freisetzung - spontane Freisetzung) x 100.

### 7. Induktion von E7-spezifischen cytotoxischen T-Lymphozyten durch CVLPs

Es wurden 11 sechs bis sechzehn Wochen alte weibliche C57BL/6 Mäuse mit 1-20 µg CVLPs ohne Adjuvans durch eine einfache s.c. Injektion immunisiert. Zwei Wochen später wurden die Milzzellen präpariert und mit HPV-16 E7 exprimierenden Transfektanten der C57BL/6 abgeleiteten Tumorzelllinie RMA (RMA-E7) als Stimulatorzellen in vitro stimuliert. Nach fünftägiger Kultivierung wurde der erste Zytotoxizitätstest durchgeführt.

Die Stimulation der Milzzellen wurde in wöchentlichen Intervallen wiederholt. Wie in Fig. 1 gezeigt, führte die Immunisierung mit 1 µg von L1ΔCE7₁₋₆₀ CVLPs, d.h. die Carboxy-terminalen 34 Aminosäuren von HPV-16 L1 wurden durch die Aminosäuren 1-60 von HPV-16 E7 ersetzt, zu einer starken E7-spezifischen CTL-Antwort.

Insgesamt wurde nach fünf Tagen eine spezifische Lyse von RMA-E7 Transfektanten zwischen 7,7% und 54,7% (Mittelwert 33,6%; E:T-Verhältnis 8:1-33:1) beobachtet. Die durchschnittliche Lyse nach der zweiten Stimulation war zwischen 35,8% und 57,8% (Mittelwert 45,3%; E:T-Verhältnis 8:1-44:1). Wenn die Mäuse nur mit 1 µg L1ΔCE7₁₋₆₀ CVLPs immunisiert wurden, wurde durchschnittlich nach 5 Tagen Kultivierungszeit eine geringere Reaktivität von E7-spezifischen CTLs beobachtet (7,7-49,5%; Mittelwert 28,5%) als nach Immunisierung mit 5 µg (17,2-44,7%; Mittelwert 33,9%) oder 20 µg (26,0-54,7%; Mittelwert 38,5). Die Milzzellen von den Kontrollmäusen (vakziniert mit HBS-Puffer) zeigten keine E7-spezifische Reaktivität nach fünf Tagen (0,3 - 8,9%, Mittelwert 3,4%, E:T-Verhältnis 4:1-9:1).

L1ΔCE7₁₋₆₀ CVLP enthält ein bekanntes H2-D^{b}-T-Zellepitop des E7-Proteins (E7₄₉₋₅₇, Feltkamp, M.C.W. et al. (1993), supra). Um die erhaltene E7-Spezifität der CTLs zu bestätigen, wurden sie daher gegen RMA-Zellen getestet, die mit dem Peptid E7₄₉₋₅₇ beladen sind. CTLs, die RMA-E7 Transfektanten erkennen, zeigten eine spezifische zytotoxische Aktivität gegen E7₄₉₋₅₇ RMA oder RMA-S Zielzellen. Die gemessene spezifische Lyse variierte zwischen 21,2-25% nach fünf Tagen (E:T-Verhältnis 8:1-15:1, s. Fig. 1). In dem durchgeführten Peptidtitrationstest wurden nach der dritten Stimulation RMA-S Zellen nach Beladung mit E7₄₉₋₅₇ bei einer Konzentration von 1 µg/ml zu 70,5% bzw. 71,4% (zwei CR-Linien wurden getestet) lysiert (E:T-Verhältnis 40:1, s. Fig. 2). Sogar wenn RMA-S-Zellen mit so wenig wie 100 pg pro ml von E7₄₉₋₅₇ Peptid versehen wurden, wurde eine spezifische Lyse von 36,2% und 47,2% erhalten. CTLs, welche mit einem Kontrollpeptid, d.h. dem Influenzavirus-Nucleoprotein-abgeleiteten Peptid mit den Aminosäuren 366-374, die ein D^{b}-CTL-Epitop repräsentieren, beladen sind, wurden nicht erkannt. Milzzellen, die aus C57BL/6-Mäusen nach Behandlung mit L1ΔCE7₁₋₅₅ CVLPs (E7-Sequenz um fünf Aminosäuren verkürzt) isoliert wurden, erkannten nicht RMA-E7-Zellen, sogar wenn die Mäuse mit einer ansteigenden Dosis bis zu 250 µg vakziniert wurden.

Aus diesen Daten wird geschlossen, daß die CTLs durch L1ΔCE7₁₋₆₀ CVLPs induziert wurden, E7-spezifisch sind und das Peptid E7₄₉₋₅₇ mit hoher Affinität erkennen. In einer FACS-Analyse von repräsentativen CTL-Linien wurden die CTLs als CD8-positiv typisiert.

### 8. Verhinderung des Wachstums eines syngenen E7-exprimierenden Tumors in C57BL/6 Mäusen

Um zu bestimmen, ob die Vaccinierung mit L1ΔCE7₁₋₆₀ CVLPs eine effektive Immunität gegen syngene Tumorzellen (TC-1, Lin, K-Y. (1996), Cancer Res. 56, 21-26) induzieren, wurden Tumorschutzexperimente durchgeführt. C57BL/6 Mäuse (3-5 pro Gruppe) erhielten 1 s.c. Injektion von 10 µg L1ΔCE7₁₋₆₀ CVLPs, 10 µg L1ΔCE7₁₋₅₅ CVLPs, 10 µg L1ΔC VLPs oder ein äquivalentes Volumen an HBS-Puffer ohne Adjuvans. Zwei Wochen nach der Immunisierung wurden den Mäusen 6 x 10⁴ TC-1 Zellen, suspendiert in 200 µl PBS, in die linke Flanke inokuliert. 2 Monate lang wurde die Tumorgröße zweimal pro Woche gemessen bis die Mäuse getötet wurden.

Die Ergebnisse aller Experimente sind in der Tabelle 1 zusammengefaßt. Die gesamte Tumoranwachesrate in den Mäusen, die mit HBS-Puffer, L1ΔC VLPs oder L1ΔCE7₁₋₅₅ CVLPs vakziniert wurden, war durchschnittlich 80,5%. Die Vaccinierung mit L1ΔC CVLPs schützte nicht gegen den Tumor, obwohl ein verzögertes Tumorwachstum beobachtet wurde (s. Fig. 3). Dagegen schützte die Vaccinierung von Mäusen mit L1ΔCE7₁₋₆₀ CVLPs gegen Tumorwachstum: nur eine Maus (1/13) entwickelte einen kleinen, langsam wachsenden Tumor nach 38 Tagen, welcher innerhalb einer zweimonatigen Periode zurückging.

Milzzellen von HBS- oder L1ΔC-VLP-vaccinierten Mäusen, welche TC-1 Tumore entwickelten, zeigten keine oder nur geringe Levels einer E7-spezifischen CTL-Antwort, unabhängig davon, ob die Mäuse Tumore entwickelt hatten oder nicht. Nach der 2. Stimulation wurde eine E7-spezifische Lyse von RMA-E7 Zielzellen zwischen 0-21% in tumortragenden Mäusen (Mittelwert 14%, E:T-Verhältnis 5:1-24:1), und zwischen 0 und 22,2% (Mittelwert 10,4%, E:T-Verhältnis 7:1-40:1) in tumorfreien Mäusen beobachtet. Im Gegensatz dazu wurden in Mäusen, welche nach Immunisierung mit CVLP L1ΔCE7₁₋₆₀ vom Tumorwachstum geschützt wurden, eine E7-spezifischen CTL-Antwort zwischen 29,1 und 49,8% (Mittelwert 36,9%, E:T-Verhältnis 11:1-24:1) nach der 2. Stimulation festgestellt.

Weiterhin wurde analysiert, ob die Vaccinierung zu einer Regression von bereits existierenden Tumoren führt. Zwei Wochen nach der Inokulation von 6 x 10⁴ TC-1-Zellen (s.c.) erhielten die Mäuse (5 pro Gruppe) eine einzige Injektion von 10 µg L1ΔCE7₁₋₆₀ CVLPs, 10 µg VLP L1ΔC oder HBS-Puffer ohne Adjuvans. In diesem Experiment (s. Fig. 4) war die Tumorentwicklung von Kontrollmäusen, die entweder mit HBS behandelt oder mit VLP L1ΔC immunisiert wurden, nur 60% (3 von jeder Gruppe, 6/10). Jedoch generierten alle Mäuse (5/5), die mit L1ΔCE7₁₋₆₀ CVLPs vakziniert wurden, eine Immunantwort gegen die etablierten Tumore und blieben zwei Monate lang nach der Tumorzellinjektion tumorfrei.

## Patentansprüche

1. Verwendung mindestens eines Fusionsproteins aus mindestens einem L1-Protein eines oder mehrerer Papillomaviren und mindestens einem C-terminal deletierten E7-Protein eines oder mehrerer Papillomaviren, bei dem 38 bis 43 Aminosäuren deletiert sind, wobei das Fusionsprotein keine Papillomavirus-unspezifischen Epitope enthält, und gegebenenfalls geeignete Zusatzund/oder Hilfsstoffe zur Herstellung eines Arzneimittels für die Anwendung am Menschen zur Induktion einer spezifischen zytotoxischen T-Zell (CTL)-Antwort einerseits zur Verhinderung von HPV-spezifischen Tumoren und andererseits zur Regression von bereits bestehenden humanen Papillomavirus (HPV)-spezifischen Tumoren.

2. Verwendung nach Anspruch 1, **dadurch gekennzeichnet, dass** der Tumor ein Larynx-, Cervix-, Penis-, Vulva- oder Analcarcinom ist.

3. Verwendung nach Anspruch 1 oder 2, **dadurch gekennzeichnet, dass** das Arzneimittel kein Adjuvans enthält.

4. Verwendung nach einem der Ansprüche 1-3, **dadurch gekennzeichnet, dass** der Zusatz- oder Hilfsstoff 0,3 bis 4 M, vorzugsweise 0,4 bis 3 M, insbesondere 0,5 bis 2 M, vor allem 1 bis 2 M eines Salzes mit einem pH-Wert von 7,3 bis 7,45, vorzugsweise 7,4 ist.

5. Verwendung nach Anspruch 4, **dadurch gekennzeichnet, dass** das Salz ein Alkali- oder Erdalkatisalz ist, vorzugsweise ein Halogenid oder Phosphat, insbesondere ein Alkalihalogenid, vor allem NaCl und/oder KCl.

6. Verwendung nach Anspruch 4 oder 5, **dadurch gekennzeichnet, dass** der pH-Wert mit einem Puffer eingestellt wird, vorzugsweise mit einem Phosphat-Puffer, Tris-Puffer, HEPES-Puffer oder MOPS-Puffer.

7. Verwendung nach einem der Ansprüche 1-6, **dadurch gekennzeichnet, dass** das L1-Protein ein deletiertes L1-Protein ist.

8. Verwendung nach Anspruch 7, **dadurch gekennzeichnet, dass** das L1-Protein ein C-terminal deletiertes L1-Protein ist.

9. Verwendung nach Anspruch 7 oder 8, **dadurch gekennzeichnet, dass** bis zu 35 Aminosäuren vom L1-Protein deletiert sind, vorzugsweise 25 bis 35 Aminosäuren, insbesondere 32 bis 34 Aminosäuren.

10. Verwendung nach einem der Ansprüche 1-9, **dadurch gekennzeichnet, dass** das Fusionsprotein in Form eines Capsids und/oder Capsomers vorliegt.

11. Verwendung nach einem der Ansprüche 1-10, **dadurch gekennzeichnet, dass** das HPV ausgewählt ist aus HPV-6, HPV-11, HPV-16, HPV-18, HPV-31, HPV-33, HPV-35, HPV-39, HPV-42, HPV-45, HPV-52 und/oder HPV-58.

12. Verwendung nach Anspruch 11 in Form einer Kombinationsvakzine, **dadurch gekennzeichnet, dass** die Papillomaviren ausgewählt sind aus HPV-16 und HPV-18; oder HPV-18, HPV-31, HPV-45 und HPV-58; oder HPV-6 und PPV-11.

13. Verwendung nach einem der Ansprüche 1-12, **dadurch gekennzeichnet, dass** das Fusionsprotein ein L1ΔCE7₁₋ₓ-Fusionsprotein, vorzugsweise in Form eines CVLP, insbesondere von HPV-16 ist, wobei x eine ganze Zahl von 55 bis einschließlich 60 bedeutet.

14. Verwendung nach Anspruch 13, **dadurch gekennzeichnet, dass** das Fusionsprotein ein L1ΔCE7₁₋₅₅- oder L1ΔCE7₁₋₆₀-Fusionsprotein ist.

## Claims

1. Use of at least one fusion protein from at least one L1 protein of one or more papillomaviruses and at least one C-terminally deleted E7 protein of one or more papillomaviruses, from 38 to 43 amino acids being deleted and the fusion protein containing no papillomavirus-unspecific epitopes, with or without suitable additive and/or excipient materials for production of a medicament for use on humans to induce a specific cytotoxic T-cell (CTL) response both to prevent HPV-specific tumours and to regress already existing HPV-specific tumours.

2. Use according to Claim 1, **characterized in that** the tumour is a carcinoma of the larynx, cervix, penis, vulva or anus.

3. Use according to Claim 1 or 2, **characterized in that** the medicament contains no adjuvant.

4. Use according to one of Claims 1-3, **characterized in that** the additive or excipient is 0.3 to 4 M, preferably 0.4 to 3 M, in particular 0.5 to 2 M, especially 1 to 2 M, of a salt having a pH of 7.3 to 7.45, preferably 7.4.

5. Use according to Claim 4, **characterized in that** the salt is an alkali metal or alkaline earth metal salt, preferably a halide or phosphate, in particular an alkali metal halide, especially NaCl and/or KCl.

6. Use according to Claim 4 or 5, **characterized in that** the pH is adjusted using a buffer, preferably using a phosphate buffer, tris buffer, HEPES buffer or MOPS buffer.

7. Use according to one of Claims 1-6, **characterized in that** the L1 protein is a deleted L1 protein.

8. Use according to Claim 7, **characterized in that** the L1 protein is a C-terminally deleted L1 protein.

9. Use according to Claim 7 or 8, **characterized in that** up to 35 amino acids are deleted from the L protein, preferably 25 to 35 amino acids, in particular 32 - 34 amino acids.

10. Use according to one of Claims 1-9, **characterized in that** the fusion protein is present in the form of a capsid and/or capsomer.

11. Use according to one of Claims 1-10, **characterized in that** the HPV is selected from HPV-6, HPV-11, HPV-16, HPV-18, HPV-31, HPV-33, HPV-35, HPV-39, HPV-42, HPV-45, HPV-52 and/or HPV-58.

12. Use according to Claim 11 in the form of a combination vaccine, **characterized in that** the papillomaviruses are selected from HPV-16 and HPV-18; or HPV-18, HPV-31, HPV-45 and HPV-58; or HPV-6 and HPV-11.

13. Use according to one of Claims 1-12, **characterized in that** the fusion protein is an L1ΔCE7₁₋ₓ fusion protein, preferably in the form of a CVLP, in particular of HPV16, x being an integer from 55 up to and including 60.

14. Use according to Claim 13, **characterized in that** the fusion protein is an L1ΔCE7₁₋₅₅ or L1ΔCE7₁₋₆₀ fusion protein.

## Revendications

1. Utilisation au moins d'une protéine de fusion composée au moins d'une protéine L1 d'un ou de plusieurs papillomavirus et d'au moins une protéine E7 délitée et terminée en C d'un ou de plusieurs papillomavirus, protéine dans laquelle 38 à 43 acides aminés sont délités, la protéine de fusion ne contenant aucune épitope non spécifique à un papillomavirus, et le cas échéant des additifs et/ou des agents auxiliaires appropriés pour la fabrication d'un médicament pour application à l'homme pour induction d'une réponse à cellule T (CTL) cytotoxique spécifique d'une part pour la prévention des tumeurs spécifiques PVH et d'autre part pour la régression de tumeurs spécifiques à papillomavirus humain (PVH) déjà déclarées.

2. Utilisation selon la revendication 1, **caractérisée en ce que** la tumeur est un carcinome du larynx, cervical, du pénis, de la vulve ou anal.

3. Utilisation selon la revendication 1 ou 2, **caractérisée en ce que** le médicament ne contient pas d'adjuvant.

4. Utilisation selon l'une des revendications 1 à 3, **caractérisée en ce que** l'additif ou l'agent auxiliaire correspond à 0,3 à 4 M, de préférence 0,4 à 3 M, en particulier 0,5 à 2 M, de préférence particulière 1 à 2 M d'un sel d'une acidité pH de 7,3 à 7,45, de préférence 7,4.

5. Utilisation selon la revendication 4, **caractérisée en ce que** le sel est un sel alcalin ou un sel alcalinoterreux, de préférence un halogénure ou un phosphate, en particulier un halogénure alcalin, de préférence particulière NaCl et/ou KCI.

6. Utilisation selon la revendication 4 ou 5, **caractérisée en ce que** l'acidité pH est ajustée à l'aide d'un tampon, de préférence un tampon de phosphate, un tampon tris, un tampon HEPES ou un tampon MOPS.

7. Utilisation selon l'une des revendications 1 à 6, **caractérisée en ce que** la protéine L1 est une protéine L1 délitée.

8. Utilisation selon la revendication 7, **caractérisée en ce que** la protéine L1 est une protéine L1 délitée et terminée en C.

9. Utilisation selon la revendication 7 ou 8, **caractérisée en ce que** 35 acides aminés au maximum de protéine L1 sont délités, de préférence 25 à 35 acides aminés, en particulier 32 à 34 acides aminés.

10. Utilisation selon l'une des revendications 1 à 9, **caractérisée en ce que** la protéine de fusion est sous forme de capside et/ou de capsomère.

11. Utilisation selon l'une des revendications 1 à 10, **caractérisée en ce que** le PVH est sélectionné parmi le PVH-6, le PVH-11, le PVH-16, le PVH-18, le PVH-31, le PVH-33, le PVH-35, le PVH-39, le PVH-42, le PVH-45, le PVH-52 et/ou le PVH-58.

12. Utilisation selon la revendication 11 sous forme de vaccin combiné, caractérisée en que les papillomavirus sont sélectionnés parmi le PVH-16 et le PVH-18 ; ou bien le PVH-18, PVH-31, le PVH-45 et le PVH-58; ou bien le PVH-6 et le PVH-11.

13. Utilisation selon l'une des revendications 1 à 12, **caractérisée en ce que** la protéine de fusion est une protéine de fusion L1ΔCE7₁₋ₓ, de préférence sous forme de CVLP, en particulier PVH-16, x correspondant à un nombre entier de 55 à 60 compris.

14. Utilisation selon la revendication 13, **caractérisée en ce que** la protéine de fusion est une protéine de fusion L1ΔCE7₁₋₅₅ ou L1ΔCE7₁₋₆₀.
